Europäisches Patentamt

European Patent Office

Office européen des brevets

(11) Publication number: **0 231 044**
**A2**

## EUROPEAN PATENT APPLICATION

(21) Application number: **87200068.2**

(22) Date of filing: **16.01.87**

(51) Int. Cl.⁴: **C 07 C 67/38**

(30) Priority: **29.01.86 GB 8602177**
**29.01.86 GB 8602178**

(43) Date of publication of application:
**05.08.87 Bulletin 87/32**

(84) Designated Contracting States:
**BE DE ES FR GB IT NL SE**

(71) Applicant: **SHELL INTERNATIONALE RESEARCH**
**MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

**Breed, Antonius Johannus Maria**
**Badhuisweg 3**
**NL-1031 CM Amsterdam (NL)**

(74) Representative: **Aalbers, Onno et al**
**P.O. Box 302**
**NL-2501 CH The Hague (NL)**

(54) Process for the preparation of a diester of a dicarboxylic acid.

(57) Process for the preparation of dicarboxylate esters by reacting an optionally substituted ethylenically or acetylenically unsaturated hydrocarbon having two carbon atoms less than said dicarboxylic acid, CO and an alcohol in the presence of a Group 8 noble metal or a compound thereof and of a quinone. The process includes the preparation of diesters of 2-butene-dioic acids derived from acetylenically unsaturated hydrocarbons.

EP 0 231 044 A2

**Description**

PROCESS FOR THE PREPARATION OF A DIESTER OF A DICARBOXYLIC ACID

The invention relates to a process for the preparation of a diester of a dicarboxylic acid. Dicarboxylic acids include 2-butenedioic acids.

It is known from U.S. patent specification 3,759,984 to prepare succinic acid and/or derivatives thereof by reacting ethylene with carbon monoxide and an alcohol in the presence of a palladium compound having a strong acid residual group, an amino acid, and a heavy metal salt while supplying oxygen to increase the reaction rate and to prolong the catalyst life. The examples of this known process show that dimethyl succinate was obtained in a low yield, calculated on starting ethylene.

It is known from US patent specification 3,755,419 to prepare 2-butenedioic acid or a derivative thereof by reacting acetylene with carbon monoxide and an alcohol in the presence of a palladium compound having a strong acid residual group, an amino acid and a heavy metal salt. The examples of this known process show that the 2-butenedioate esters were obtained in a low yield, calculated on starting acetylene. Moreover, an appreciable part of the esters formed consisted of the trans configuration, dimethyl fumarate.

It is an object of the present invention to prepare diesters of dicarboxylic acids in a very high yield, including diesters of a 2-butenedioic acid in a high yield and in the cis-configuration.

Accordingly, the invention provides a process for the preparation of a diester of a dicarboxylic acid which process comprises reacting an optionally substituted ethylenically or acetylenically unsaturated hydrocarbon having two carbon atoms per molecule less than said dicarboxylic acid, carbon monoxide and an alcohol in the presence of:-
   a) a noble metal of Group 8 of the Periodic Table of the Elements and/or a compound thereof, and
   b) a quinone.

The noble metals which are used in the process according to the present invention are platinum, rhodium, ruthenium, palladium, iridium and/or osmium. These metals may be used in metallic form or as compounds. Mixtures of compounds of the same or different such noble metals or mixtures of such noble metals in metallic form may be used. The noble metals may be used as finely divided metals, not supported on a carrier, or supported on a carrier, for example on activated carbon, pumice or graphite. The present process is preferably carried out in the presence of palladium and/or a compound of palladium. Very good results have been obtained with compounds of palladium. Examples of suitable compounds of Group 8 noble metals are salts, such as nitrates, sulphates, halides, (fluorides, chlorides, bromides and iodides) and carboxylates. Among the carboxylates salts of alkanoic acids having not more than 12 carbon atoms per molecule are preferred, particularly the Pd (II) salts. Palladium (II) acetate is most preferred.

Further examples of suitable palladium compounds are palladium complexes such as bis(2,4-pentanedionato)palladium, bis(picolinato)palladium, tetrakis(triphenylphosphine)palladium, tris(dibenzylideneacetone)dipalladium, bis(triphenylphosphine)(1,4-benzoquinone)palladium, tetrakisacetonitrilepalladium tetrafluoroborate, bis(tri-o-tolylphosphine)palladium acetate, bis(triphenylphosphine)palladium sulphate, palladium olefin complexes, for instance di-$\mu$-chloro-dichlorobis(ethylene)dipalladium($[Pd.C_2H_4.Cl_2]_2$), and di-$\mu$-chlorodichlorobis(propylene)dipalladium($[Pd.C_3H_6.Cl_2]_2$), and palladiumhydride complexes. Palladium may be used in complex combination with phosphites, such as triphenyl phosphite or tributyl phosphite.

The quinone applied in the process according to the present invention may be an ortho-quinone or a para-quinone and may be, for example, a benzoquinone, a naphthoquinone, an anthraquinone or a chrysenequinone. Preference is given to optionally substituted benzoquinones, particularly to p-benzoquinones. According to a preferred embodiment of the present invention halogen-substituted p-benzoquinones are present, very high yields of diesters of dicarboxylic acids being obtained. In this embodiment one or more fluorine, chlorine, bromine and/or iodine atoms are attached to the aromatic nucleus of the p-benzoquinone. Examples of such quinones are 2-iodo-, 2-bromo-, 2-chloro- and 2-fluoro-p-benzoquinone, 2,6-diiodo-, 2,6-dibromo-, 2,6-dichloro- and 2,6-difluoro-p-benzoquinone, 2,3,6-triiodo-, 2,3,6-tribromo-, 2,3,6-trichloro- and 2,3,6-trifluoro-p-benzoquinone. Tetrahalo-p-benzoquinones are preferred, particularly tetrachloro-p-benzoquinone (also referred to as "chloranil"). According to another preferred embodiment unsubstituted p-benzoquinone is applied in the present process. Further examples of suitable quinones are 9,10-anthraquinone, 1,4-naphthoquinone, 5,6-chrysenequinone and alkyl-substituted p-benzoquinones such as 2-methyl-p-benzoquinone and 2,6-dimethyl-p-benzoquinone. Mixtures of quinones may be present, for example of p-benzoquinone and a halogen-substituted p-benzoquinone.

It has been found that the yield of diesters of dicarboxylic acids is further enhanced by carrying out the present process in the presence of a redox agent. Redox agents are well known in the art. It is generally a compound of copper, iron, vanadium, cobalt or manganese. Mixtures of such compounds may be used. These five metals are preferably used in the form of salts, such as chlorides, nitrates, sulphates, carboxylates, preferably those carboxylates having not more than 12 carbon atoms per molecule, perchlorates and sulphonates, for example benzenesulphonates and p-tosylates. Among the metal compounds cupric compounds are preferred, particularly cupric tosylate and cupric perchlorate. Further examples of suitable redox agents are cobalt (II) complexes of organic ligands, cupric acetate, cupric butyrate, cupric chloride, cupric bromide, ferrous propionate, ferric acetate, ferric chloride, ferric bromide and cobalt (II) acetate. Very high yields of diesters of dicarboxylic acids have been obtained with ferrous perchlorate.

2

It has, moreover, been found that the yield of diesters of 2-butenedioic acids is further enhanced by carrying out the present process in the presence of a redox agent comprising a copper, iron, vanadium, cobalt or manganese salt of methanesulphonic acid, a substituted methanesulphonic acid, sulphuric acid or perchloric acid. Mixtures of such salts may be used, for example of copper and iron salts. Among the substituted methanesulphonates preference is given to halomethanesulphonates of said metals, i.e. mono-, di- and trihalomethanesulphonates, "halo" referring to fluoro, chloro, bromo or iodo. Preference is given to trifluoromethanesulphonates, particularly to cupric trifluoromethanesulphonate. Other examples of substituents on methanesulphonates are methyl, ethyl, propyl and isopropyl groups. Cupric and ferrous perchlorate are two other preferred redox agents.

The process according to the present invention may be carried out using a molar ratio noble metal of Group 8 and/or a compound thereof to optionally substituted ethylenically or acetylenically unsaturated hydrocarbon which is not critical and may vary within wide ranges. This molar ratio is suitably in the range of from $10^{-2}$ to $10^{-6}$.

The process according to the present invention may be carried out using a molar ratio redox agent to noble metal of Group 8 and/or a compound thereof which is not critical and may vary within wide ranges. This molar ratio is suitably in the range of from 0.5 to 1000 and preferably from 1 to 200.

The process according to the present invention results in the formation of a diester of a dicarboxylic acid or of a 2-butenedioic acid and of a hydroquinone. The hydroquinone may be isolated from the reaction mixture and, if desired, purified. The isolated and optionally purified hydroquinone may be used for any suitable purpose but is preferably oxidized in a suitable manner to the corresponding quinone which quinone is preferably used in the process according to the present invention.

It has, furthermore, been found that the yield of diesters of dicarboxylic acids is further enhanced by carrying out the present process in the presence of molecular oxygen. The molecular oxygen may be supplied, for example, as pure oxygen, in air enriched with oxygen, in air or diluted with an inert gas such as argon. The oxygen is supplied using a molar ratio molecular oxygen to carbon monoxide which is not critical and may vary within wide ranges, preferably in the range of from 1 : 3 to 1 : 6, the stoichiometric ratio being 1 : 4. The oxygen may be supplied in one portion but may, for safety reasons, be supplied in portions.

The process according to the present invention can be carried out using a molar ratio quinone to optionally substituted ethylenically or acetylenically unsaturated hydrocarbon which is not critical and which may vary within wide limits. This molar ratio may vary, for example, in the range of from 0.001 to 10, preferably from 0.001 to 5.

The process according to the present invention can be carried out using a molar ratio carbon monoxide to optionally substituted ethylenically or acetylenically unsaturated hydrocarbon which is not critical and may vary within wide limits, preferably in the range of from 0.5 : 1 to 10 : 1 and particularly from 1 : 1 to 3 : 1, the stoichiometric ratio being 2.

The process according to the present invention can be carried out in wide ranges of temperature and pressure, preferably in the range of from 20 °C to 200 °C, more preferably from 50 °C to 125 °C, and at a pressure in the range of from 1 to 200 bar preferably in the range of from 5 to 200 bar, more preferably from 10 to 100 bar.

The alcohol can be applied in a molar ratio alcohol to optionally substituted ethylenically or acetylenically unsaturated hydrocarbon which is not critical and may vary within wide limits. This molar ratio is suitably at least the stoichiometric molar ratio which is 2 and may be, if desired, in the range of from 2 to 1000.

The process according to the present invention can be carried out in the absence or, which is preferred, in the presence of a solvent which does not inhibit the reaction. The alcohol which is used as a reactant may be used as a solvent. Very good results have been obtained with ethers. Examples of ethers are methyl ethyl ether, diethyl ether, dipropyl ether, tetrahydrofuran, dimethyl ether of diethylene glycol (also referred to as "diglyme"), methyl tert.-butyl ether, dichloroethyl ether, ethyl phenyl ether, diethylene glycol diethyl ether and 1,4-dioxane. Other examples of suitable solvents are halogenated hydrocarbons such as chloroform, chlorobenzene, carbon tetrachloride and perfluoroalkanes; esters such as the methyl and ethyl esters of formic acid, acetic acid, adipic acid, succinic acid, propionic acid, oxalic acid and benzoic acid; sulphones such as dimethyl sulphone, methyl butyl sulphone and tetrahydrothiophone 1,1-dioxide (also referred to as "sulfolane"); aromatic hydrocarbons such as benzene, toluene and the three xylenes; cycloalkanes such as cyclohexane; nitrobenzene.

The process according to the present invention is suitably carried out with an optionally substituted ethylenically or acetylenically unsaturated hydrocarbon carrying a hydrogen atom to at least one of the doublebonded carbon atoms or triple-bonded carbon atoms. Preference is given to optionally substituted alkenes having in the range of from 2 to 20 and particularly 2 to 10 carbon atoms per molecule or an optionally substituted cycloalkene having in the range of from 3 to 20 carbon atoms per molecule. Very good results have been obtained with ethylene. Other examples of suitable ethylenically unsaturated hydrocarbons are propene, 1-butene, 2-butene, isobutene, 1-pentene, 2-pentene, the n-hexenes, the n-heptenes, the n-octenes, the n-nonenes, the n-decenes, the n-undecenes, the n-dodecenes, the n-eicosenes, 2-methyl-1-butene, 2-methyl-2-butene, 2-ethyl-1-butene, 2-methyl-1-pentene, 2-ethyl-3-hexene, 2-ethyl-1-octene, styrene, α-methylstyrene and allylbenzene. Further examples are cycloalkenes such as cyclopentene, cyclohexene, cycloheptene, cyclooctene; indene and phenaline. Preference is given to optionally substituted alkynes having in the range of from 2 to 20 and particularly 2 to 10 carbon atoms per molecule. Very good results have been obtained with ethyne, also referred to as "acetylene". Other examples of suitable acetylenically unsaturated hydrocarbons are propyne, 1-butyne, 2-butyne, 1-pentyne, 2-pentyne, the n-hexynes, the n-heptynes, the n-octynes, the n-nonynes, the n-decynes, the n-undecynes, the n-dodecynes, the n-eicosynes, 3-methyl-1-butyne, 3-methyl-1-pentyne, 2-ethyl-3-hexyne, 3-ethyl-1-octyne, phenylethyne, benzylethyne and cyclohexylethyne.

A wide variety of alcohols may be used in the process according to the present invention; the alcohol may be mono- or polyhydric, may be primary, secondary or tertiary and may be aliphatic, cycloaliphatic or aromatic. Monohydric alcohols having in the range of from 1 to 20 carbon atoms per molecule and, particularly, alkanols, are preferred. Very good results have been obtained with methanol. Other examples of suitable alcohols are ethanol, propanol, butanol, pentanol, hexanol, heptanol, octanol, nonanol, decanol, ethylene glycol, diethylene glycol, propylene glycol, ethylene glycol monoalkyl ethers (the alkyl group having up to, for example, 10 carbon atoms), 1,3-butanediol, cyclohexanol, phenol, benzyl alcohol, 2-naphthol and 2-phenanthrol.

The acid portion of the diester obtained according to the present invention is derived from the optionally substituted ethylenically or acetylenically unsaturated hydrocarbon and the alcohol portion of the diester is derived from the alcohol. Accordingly, ethylene, methanol and carbon monoxide are converted into dimethyl succinate; propene, ethanol and carbon monoxide into diethyl 2-methylbutanedioate; 2-butene, methanol and carbon monoxide into dimethyl 2,3-dimethylbutanedioate; isobutene, methanol and carbon monoxide into dimethyl 3-methylpentanedioate; ethyne, methanol and carbon monoxide into dimethyl maleate and dimethyl fumarate; propyne, ethanol and carbon monoxide into diethyl 2-methyl-2-butenedioate; and 1-butyne, methanol and carbon monoxide into dimethyl 2-ethyl-2-butenedioate.

The process according to the invention can be carried out batchwise, semi-continuously or continuously. The reaction time varies in relation to the temperature used and is usually between 0.5 and 20 hours. The following Examples further illustrate the invention.

Examples 1-10

The Examples 1-10 were carried out in a 300 ml autoclave made of Hastelloy C ("Hastelloy" is a trade name) provided with a magnetically driven stirrer. In all experiments the autoclave was charged with palladium(II) acetate (0.1 mmol), methanol (10 ml, 247 mmol) and diglyme (50 ml). The autoclave was further charged with a quinone and a redox agent (except in Example 10), flushed with carbon monoxide, charged with carbon monoxide, ethylene and, in four Examples with air, all this as detailed in Table I hereinafter, heated to the temperature stated in Table I and kept at this temperature for the time also stated in Table I. Then, the amounts of dimethyl succinate and quinone (or oxygen where air has been used) in the reaction mixture were determined. The only by-products which could be detected were methyl acrylate and methyl 3-methoxypropionate, which were formed in a total amount of less than 2 %mol, calculated on dimethyl succinate. The table I also presents the conversion of the quinone and the yield of dimethyl succinate in mmol.

TABLE I

| Example | Quinone mmol | Redox agent mmol | Pressure, bar, | | | Reaction time, h | Temperature, °C | Conversion quinone ($O_2$ in air), % | Yield of dimethyl succinate, mmol |
|---|---|---|---|---|---|---|---|---|---|
| | | | $C_2H_4$ | CO | air | | | | |
| 1 | chloranil 50 | Cu(tosylate)$_2$ 2.0 | 10 | 20 | 0 | 2 | 90 | 75 | 37.5 |
| 2 | ditto | ditto | 10 | 20 | 20 | 5 | 80 | (100) | 55 |
| 3 | p-benzoquinone 50 | ditto | 10 | 20 | 0 | 5 | 90 | 38 | 19 |
| 4 | chloranil 50 | CuCl$_2$ 2.0 | 10 | 20 | 0 | 0.75 | 90 | 60 | 30 |
| 5 | ditto | VOSO$_4$ 2.0 | 10 | 20 | 0 | 1 | 90 | 73 | 36.5 |
| 6 | ditto | CuCl$_2$ 2.0 | 20 | 20 | 0 | 1.5 | 90 | 78 | 39 |
| 7 | ditto | none | 10 | 20 | 20 | 5 | 80 | (100) | 43 |
| 8 | ditto | Fe(ClO$_4$)$_2$ 2.0 | 10 | 20 | 0 | 1 | 90 | 73 | 36.5 |
| 9 | ditto | MnCl$_2$ 2.0 | 10 | 20 | 20 | 5 | 80 | (100) | 40 |
| 10 | ditto | Cu(CF$_3$SO$_3$H)$_2$ 2.0 | 10 | 20 | 20 | 2.5 | 90 | (100) | 70 |

### Example 11

An experiment was carried out in the manner of Examples 1-10, starting from palladium(II) acetate (0.1 mmol), methanol (10 ml, 247 mmol), diglyme (50 ml), chloranil (50 mmol), cupric tosylate (2 mmol), 1-octene (20 ml, 127 mmol), carbon monoxide (20 bar) and air (20 bar). The autoclave was kept for 5h at a temperature of 80 °C. The conversions of the molecular oxygen and the 1-octene were 100% and 55%, respectively, with a selectivity to dimethyl $C_6$-butanedioates of 79%(the content of n-hexylbutanedioate being 85%), to methyl hydrogen $C_6$-butanedioates of 16% and to methyl n-nonenoates of 5%. The selectivity to a certain compound, expressed in a percentage, is defined herein as $100 \times a/b$ in which "a" is the amount of starting ethylenically unsaturated compound that has been converted into that certain compound and "b" is the total amount of starting ethylenically unsaturated compound that has been converted.

### Example 12

The experiment of Example 11 was repeated with the difference that 20 ml of 1-dodecene instead of 20 ml of 1-octene were used. The conversions of the molecular oxygen and 1-dodecene were 100% and 64%, respectively, with a selectivity to dimethyl $C_{10}$-butanedioate of 83%(the content of n-decylbutanedioate being 85%), to methyl hydrogen $C_{10}$-butanedioates of 12% and to methyl n-tridecenoates of 5%.

### Comparative Example A

An experiment was carried out which differed from Example 2 only in that no quinone was present. No reaction was observed after 1 h at 90 °C. Subsequently, after 3 h at 100 °C only 5 mmol of dimethyl succinate had been formed.

### Example 13

An experiment was carried out in the manner of Example 1, starting from palladium(II) acetate (0.1 mmol), methanol (10 ml, 247 mmol), diglyme (50 ml), chloranil (50 mmol), cupric tosylate (2 mmol), isobutene (5 ml, 49 mmol), carbon monoxide (20 bar) and air (20 bar). The autoclave was kept for 5 h at a temperature of 80 °C.

Dimethyl 3-methylpentanedioate and methyl 3-methyl-3-butenoate were formed in a yield of 20 and 33%, respectively.

### Example 14

The experiment of Example 13 was repeated with the difference that cupric chloride (2 mmol) instead of cupric tosylate (2 mmol) were used.

Dimethyl 3-methylpentanedioate and methyl 3-methyl-3-butenoate were formed in a yield of 47 and 6%, respectively.

### Examples 15-20

The Examples 15-20 were carried out in a 300 ml autoclave made of Hastelloy C ("Hastelloy" is a trade name) provided with a magnetically driven stirrer. In all experiments the autoclave charged with palladium(II) acetate (0.1 mmol), methanol (10 ml, 247 mmol) and diglyme (40 ml). The autoclave was further charged with a quinone and a redox agent (except in Example 16), as detailed in Table II hereinafter, flushed with carbon monoxide, charged with carbon monoxide until a partial pressure thereof of 15 bar was obtained and charged with acetylene until a partial pressure thereof of 2.4 bar was obtained, which is equivalent to about 35 mmol of acetylene. Then, the autoclave was heated to the temperature stated in Table II and kept at this temperature for the time stated in Table II. At the end of this period the amounts of dimethyl maleate, dimethyl fumarate and dimethyl 2,4-hexadienoate were determined mass spectrometrically; from these amounts the yields calculated on acetylene, presented in Table II were determined. Traces of methyl 3-methoxyacrylate and of methyl propynoate were observed in the Examples 15-19.

In Example 20 methyl 3-methoxyacrylate was formed in a yield of 40%.

6

TABLE II

| Example | Quinone mmol | Redox agent mmol | Reaction time, h | temp., °C | Yield, %, of dimethyl maleate | fumarate, | 2,4-hexadienote |
|---|---|---|---|---|---|---|---|
| 15 | chloranil 50 | cupric perchlorate 2 | 1 | 65 | 55 | 2 | 11 |
| 16 | ditto | none | 5 | 80 | 35 | 12 | 0 |
| 17 | p-benzoquinone 50 | cupric perchlorate 2 | 1 | 65 | 37 | 1.4 | 11 |
| 18 | chloranil 50 | ferrous perchlorate 4 | 5 | 80 | 77 | 9 | 0 |
| 19 | ditto | vanadyl sulphate 2 | 5 | 80 | 71 | 13 | 6 |
| 20 | ditto | cupric trifluoro-methanesulphonate 2 | 1 | 90 | 45 | 3 | 2 |

## Claims

1. Process for the preparation of a diester of a dicarboxylic acid which comprises reacting an optionally substituted ethylenically or acetylenically unsaturated hydrocarbon having two carbon atoms per molecule less than said dicarboxylic acid, carbon monoxide and an alcohol in the presence of:-
   a) a noble metal of Group 8 of the Periodic Table of the Elements and/or a compound thereof, and
   b) a quinone.

2. Process as claimed in claim 1 which is carried out in the presence of palladium and/or a compound of palladium.

3. Process as claimed in claim 1 which is carried out in the presence of a palladium compound.

4. Process as claimed in claim 2 or 3 in which said palladium compound is a Pd(II) salt of an alkanoic acid having not more than 12 carbon atoms per molecule.

5. Process as claimed in claim 4 which is carried out in the presence of palladium(II) acetate.

6. Process as claimed in any one of the preceding claims in which the quinone is an optionally substituted benzoquinone.

7. Process as claimed in claim 6 in which the optionally substituted benzoquinone is a p-benzoquinone.

8. Process as claimed in claim 7 in which the p-benzoquinone is a halogen-substituted p-benzoquinone.

9. Process as claimed in claim 8 in which the p-benzoquinone is tetrachloro-p-benzoquinone.

10. Process as claimed in claim 7 in which the p-benzoquinone is unsubstituted p-benzoquinone.

11. Process as claimed in any one of the preceding claims which is carried out in the presence of a redox agent.

12. Process as claimed in claim 11 in which said redox agent comprises a compound of copper, iron, vanadium, cobalt and/or manganese.

13. Process as claimed in claim 12 in which said redox agent comprises a perchlorate, a sulphate, a tosylate or a halomethanesulphonate.

14. Process as claimed in claim 13 in which said redox agent comprises a trifluoromethane sulphonate.

15. Process as claimed in claim 12 or 13 in which said redox agent is ferrous or cupric perchlorate or tosylate.

16. Process as claimed in any one of the preceding claims in which a molar ratio noble metal of Group 8 and/or a compound thereof to optionally substituted ethylenically or acetylenically unsaturated hydrocarbon in the range of from $10^{-2}$ to $10^{-6}$ is used.

17. Process as claimed in any one of claims 11 to 16 in which a molar ratio redox agent to noble metal of Group 8 and/or a compound thereof in the range of from 1 to 200 is used.

18. Process as claimed in any one of the preceding claims which is carried out in the presence of molecular oxygen.

19. Process as claimed in claim 18 in which a molar ratio molecular oxygen to carbon monoxide in the range of from 1 : 3 to 1 : 6 is applied.

20. Process as claimed in any one of the preceding claims in which a molar ratio carbon monoxide to optionally substituted ethylenically or acetylenically unsaturated hydrocarbon in the range of from 0.5 : 1 to 10 : 1 is applied.

21. Process as claimed in claim 20 in which said molar ratio is in the range of from 1 : 1 to 3 : 1.

22. Process as claimed in any one of the preceding claims which is carried out in the presence of a solvent.

23. Process as claimed in claim 22 in which the solvent comprises an ether.

24. Process as claimed in any one of the preceding claims wherein a pressure is maintained in the range of from 5 to 200 bar.

25. Process as claimed in any one of the preceding claims wherein a temperature is maintained in the range of from 20 °C to 200 °C.

26. Process as claimed in any one of the preceding claims in which the optionally substituted ethylenically unsaturated hydrocarbon is an optionally substituted alkene having in the range of from 2 to 20 carbon atoms per molecule or an optionally substituted cycloalkene having in the range of from 3 to 20 carbon atoms per molecule.

27. Process as claimed in claim 26 in which the optionally substituted ethylenically unsaturated hydrocarbon is an optionally substituted alkene having in the range of from 2 to 10 carbon atoms per molecule.

28. Process as claimed in claim 27 in which ethylene is used as the starting ethylenically unsaturated hydrocarbon.

29. Process as claimed in any one of the claims 1-25, in which the optionally substituted acetylenically unsaturated hydrocarbon is an optionally substituted alkyne having in the range of from 2 to 20 carbon atoms per molecule.

30. Process as claimed in claim 29, in which the optionally substituted alkyne has 2 to 10 carbon atoms.

31. Process as claimed in claim 30, in which ethyne is used as the starting acetylenically unsaturated hydrocarbon.

32. Process as claimed in any one of the preceding claims in which the alcohol is monohydric and has in the range of from 1 to 20 carbon atoms per molecule.

33. Process as claimed in claim 32 in which the alcohol is an alkanol.

34. Process as claimed in claim 33 in which the alkanol is methanol.